# EUROPEAN PATENT SPECIFICATION

(11) **EP 3 706 101 B1**
(45) Date of publication and mention of the grant of the patent: **05.10.2022**
(21) Application number: 20160768.6
(22) Date of filing: 03.03.2020
(51) Int. Cl.: G09B 23/28

(54) **LOW COST DUAL CONSOLE TRAINING SYSTEM FOR ROBOTIC SURGICAL SYSTEM OR ROBOTIC SURGICAL SIMULATOR**
KOSTENGÜNSTIGES DOPPELKONSOLENTRAININGSSYSTEM FÜR CHIRURGISCHE ROBOTERSYSTEME ODER CHIRURGISCHE ROBOTERSIMULATOREN
SYSTÈME D'ENTRAÎNEMENT À DOUBLE CONSOLE À FAIBLE COÛT POUR SYSTÈME CHIRURGICAL ROBOTIQUE OU SIMULATEUR CHIRURGICAL ROBOTIQUE

(30) Priority: 04.03.2019 US 201962813413 P; 21.02.2020 US 202016797004
(43) Date of publication of application: 09.09.2020
(73) Proprietor: Covidien LP, Mansfield, MA 02048 (US)
(72) Inventor: MEGLAN, Dwight, Westwood, MA Massachusetts 02090 (US)
(74) Representative: Maschio, Antonio

(56) References cited:
- WO-A1-2017/210098
- WO-A1-2018/144654
- US-A1- 2008 221 590
- US-A1- 2014 276 944

## Description

### CROSS-REFERENCE TO RELATED APPLICATIONS

This application claims the benefit of and priority to U.S. Provisional Patent Application Serial No. 62/813,413 filed March 4, 2019,

### BACKGROUND

Robotic surgical systems have been used in minimally invasive medical procedures. During a medical procedure, the robotic surgical system is controlled by a surgeon interfacing with a user interface. The user interface allows the surgeon to manipulate an end effector of a surgical instrument that acts on a patient. The user interface includes an input controller or handle that is moveable by the surgeon to control the robotic surgical system.

Different robotic surgical systems exist in the market each with different controls and displays. As a surgeon moves from one robotic surgical system to another, the surgeon must familiarize themselves with the controls and the displays of the particular robotic surgical system. In addition, during a surgical procedure, each system may have different alerts or alarms to indicate a condition of the robotic surgical system or the patient which may delay recognition of the alarm to a surgeon not familiar with the particular robotic surgical system.

As a training surgeon learns, becomes familiar with, or trains to use a robotic surgical system, the training surgeon may use a dual console user interface with an experienced surgeon to control the surgical robot. (WO2017210098).

Generally, dual console user interfaces are configured to provide handoff control between an experienced or instructor surgeon and the training surgeon. Traditional dual console user interfaces are expensive and necessitate a significant amount of space as two full sized user interfaces are required.

### SUMMARY

The invention relates to a dual user console for a robotic surgical system as defined in independent claim 1 and to a method of controlling a surgical robot as defined in independent method claim 12.

Such surgical system requires less space than a traditional dual console user interface and/or allows for shared control of the surgical robot. The shared control may be particularly useful during the training of a surgeon as the trainee can feel the same sensations that the experienced surgeon is feeling during the surgical procedure.

### BRIEF DESCRIPTION OF THE DRAWINGS

Various aspects of the present disclosure are described hereinbelow with reference to the drawings, which are incorporated in and constitute a part of this specification, wherein:
FIG. 1 is a schematic view of a robotic surgical system in accordance with the present disclosure including a dual user consoles, a surgical robot, and a shared control system;
FIG. 2 is a perspective view of a control arm of one of the user consoles of FIG. 1;
FIG. 3 a top view of an actuator of the control arm of FIG. 2;
FIGS. 4A and 4B are cross-sectional views of taken along the section line 4-4 of FIG. 2;
FIG. 5 is a schematic view of the dual user consoles of FIG. 1 with another shared control system provided in accordance with the present disclosure; and
FIG. 6 is a schematic view of the dual user consoles of FIG. 1 with another shared control system provided in accordance with the present disclosure.

### DETAILED DESCRIPTION

Embodiments of the present disclosure are now described in detail with reference to the drawings in which like reference numerals designate identical or corresponding elements in each of the several views. As used herein, the term "clinician" refers to a doctor, a surgeon, a nurse, or any other care provider and may include support personnel. Throughout this description, the term "proximal" refers to the portion of the device or component thereof that is closer to the clinician and the term "distal" refers to the portion of the device or component thereof that is farther from the clinician.

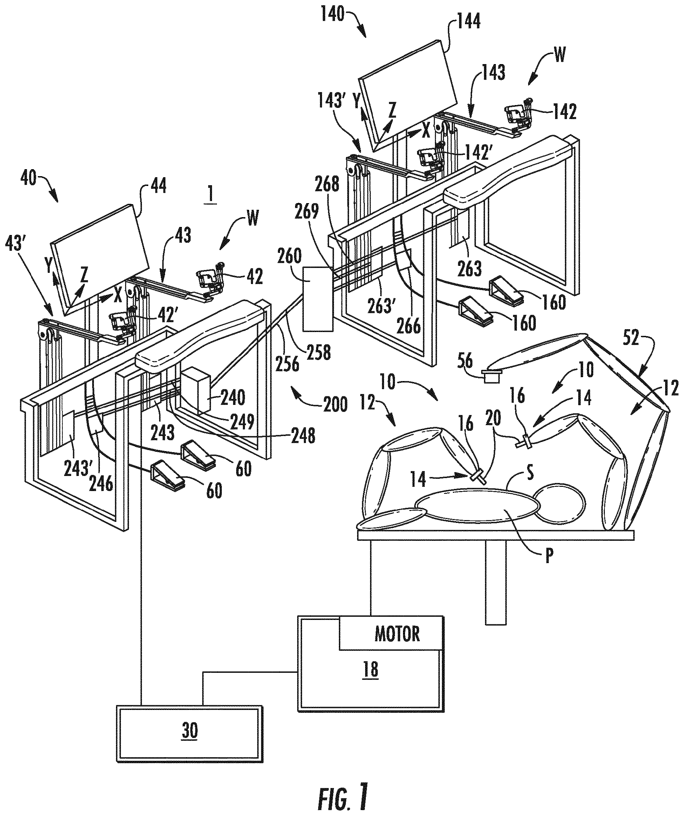

Referring to FIG. 1, a robotic surgical system 1 in accordance with the present disclosure is shown generally as a surgical robot 10, a processing unit 30, a master user console 40, and a slave user console 140. The surgical robot 10 generally includes linkages 12 and a robot base 18. The linkages 12 moveably support an end effector or tool 20 which is configured to act on tissue. The linkages 12 may be in the form of arms each having an end 14 that supports the end effector or tool 20 which is configured to act on tissue. In addition, the ends 14 of the linkages 12 may include an imaging device 16 for imaging a surgical site "S". The master user console 40 is in communication with robot base 18 through the processing unit 30.

The master user console 40 includes a display device 44 which is configured to display three-dimensional images. The display device 44 displays three-dimensional images of the surgical site "S" which may include data captured by imaging devices 16 positioned on the ends 14 of the linkages 12 and/or include data captured by imaging devices that are positioned about the surgical theater (e.g., an imaging device positioned within the surgical site "S", an imaging device positioned adjacent the patient "P", imaging device 56 positioned at a distal end of an imaging arm 52). The imaging devices (e.g., imaging devices 16, 56) may capture visual images, infra-red images, ultrasound images, X-ray images, thermal images, and/or any other known real-time images of the surgical site "S". The imaging devices transmit captured imaging data to the processing unit 30 which creates three-dimensional images of the surgical site "S" in real-time from the imaging data and transmits the three-dimensional images to the display device 44 for display.

The master user console 40 also includes input handles 42, 42' which are supported on control arms 43, 43' which allow a clinician to manipulate the surgical robot 10 (e.g., move the linkages 12, the ends 14 of the linkages 12, and/or the tools 20). Each of the input handles 42, 42' is in communication with the processing unit 30 to transmit control signals thereto and to receive feedback signals therefrom. Additionally or alternatively, each of the input handles 42, 42' may include input devices (not explicitly shown) which allow the surgeon to manipulate (e.g., clamp, grasp, fire, open, close, rotate, thrust, slice, etc.) the tools 20 supported at the ends 14 of the linkages 12.

Each of the input handles 42, 42' is moveable through a predefined workspace to move the ends 14 of the linkages 12, e.g., tools 20, within a surgical site "S". The three-dimensional images on the display device 44 are orientated such that the movement of the input handles 42, 42' moves the ends 14 of the linkages 12 as viewed on the display device 44. The three-dimensional images remain stationary while movement of the input handles 42, 42' is scaled to movement of the ends 14 of the linkages 12 within the three-dimensional images. To maintain an orientation of the three-dimensional images, kinematic mapping of the input handles 42, 42' is based on a camera orientation relative to an orientation of the ends 14 of the linkages 12. The orientation of the three-dimensional images on the display device 44 may be mirrored or rotated relative to the view captured by the imaging devices 16, 56. In addition, the size of the three-dimensional images on the display device 44 may be scaled to be larger or smaller than the actual structures of the surgical site permitting a clinician to have a better view of structures within the surgical site "S". As the input handles 42, 42' are moved, the tools 20 are moved within the surgical site "S" as detailed below. Movement of the tools 20 may also include movement of the ends 14 of the linkages 12 which support the tools 20.

For a detailed discussion of the construction and operation of a robotic surgical system 1, reference may be made to U.S. Patent No. 8,828,023,

The master user console 40 further includes one or more foot pedals 60 that can be used to control various aspects of the robotic surgical system 1. For example, the foot pedal 60 may be selectively associated with an input handle, e.g., input handle 42, to actuate a tool 20 associated with the respective input handle. Additionally or alternatively, the foot pedal 60 may be associated with a camera, e.g., camera 56, to move the camera about the surgical site "S". For a detailed discussion of suitable foot pedals, reference may be made to U.S. Provisional Patent Application Serial No. 62/510,502, filed May 24, 2017, entitled "PEDAL CONTROL FOR ROBOTIC SURGICAL SYSTEMS," and U.S. Provisional Patent Application Serial No. 62/566,100, filed September 8, 2017, entitled "HIGH PRECISION INSTRUMENT CONTROL MODE FOR ROBOTIC SURGICAL SYSTEMS,"

The slave user console 140 is similar to the master user console 40 detailed above and only the differences will be detailed herein for brevity. The slave user console 140 is in communication with the master user console 40 via a shared control system 200 such that the controls of the slave user console 140 are not in direct communication with the processing unit 30. It is contemplated that while the controls of the slave user console 140 are not in direct communication with the processing unit 30 that some elements, e.g., warning lights, indicator lights, and other audible or visual feedback elements, of the slave user console 140 may be in direct communication with the processing unit 30. In addition, the display 144 of the slave user console 140 may be in direct communication with the processing unit 30 to receive images from the processing unit 30 and to send signals to the processing unit 30.

As detailed above and shown in FIG. 1, the master and slave user interfaces 40, 140 are in operable communication with the surgical robot 10 to perform a surgical procedure on a patient "P"; however, it is envisioned that the master and slave user interfaces 40, 140 may be in operable communication with a surgical simulator (not shown) to virtually actuate a robot system and/or tool in a simulated environment. For example, the robotic surgical system 1 may have a first mode where the master and slave user interfaces 40, 140 are coupled to actuate the surgical robot 10 and a second mode where the master and slave user interfaces 40, 140 are coupled to the surgical simulator to virtually actuate a surgical robot. The surgical simulator may be a standalone unit or be integrated into the processing unit 30. The surgical simulator virtually responds to a clinicians interfacing with the master and slave user interfaces 40, 140 by providing visual, audible, force, and/or haptic feedback to clinicians through the master and slave user interfaces 40, 140. For example, as a clinician interfaces with the input device handles 42, 142, the surgical simulator moves representative tools that are virtually acting on tissue at a simulated surgical site. It is envisioned that the surgical simulator may allow a clinician to practice a surgical procedure before performing the surgical procedure on a patient. In addition, the surgical simulator may be used to train a clinician on a surgical procedure. Further, the surgical simulator may simulate "complications" during a proposed surgical procedure to permit a clinician to plan a surgical procedure.

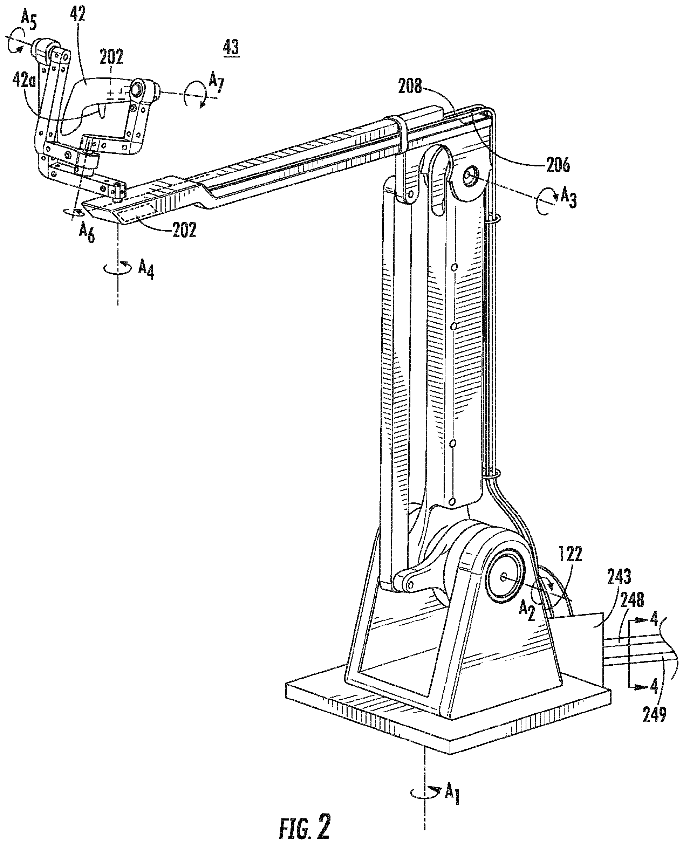

Referring now to FIGS. 1 and 2, the shared control system 200 is provided in accordance with the present disclosure to synchronize the controls of the master user console 40 and the controls of the slave user console 140. It will be appreciated, that the term master and slave are used with respect to how the user consoles 40, 140 are related to the connection with the processing unit 30 and that movements of either one of the user consoles 40, 140 may be translated to the other user console 40, 140 as detailed below.

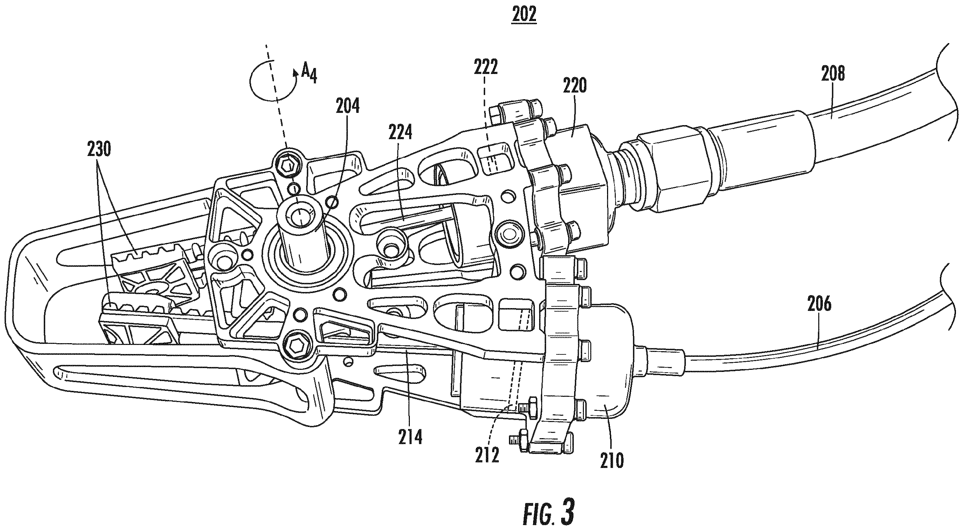

With additional reference to FIG. 3, the shared control system 200 includes an actuator 202 at each joint, e.g., fourth axis of rotation A₄, of the control arm 43. The actuators 202 are hybrid actuators including a spindle 204, a pneumatic line 206, and a hydraulic line 208. The pneumatic line 206 functions as a preloaded spring and damping system for rotation of the spindle 204. The hydraulic line 208 provides torque to rotate the spindle 204. The spindles 204 of the actuators 202 are capable of being driven by the pneumatic and hydraulic lines 206, 208 and are capable of being back driven.

The pneumatic line 206 is coupled to a cylinder 210 to rotate the spindle 204. The cylinder 210 includes a diaphragm 212 that is coupled to a rod 214. The diaphragm 212 moves back and forth within the cylinder to rotate the spindle 204. The rod 214 is coupled to a gear system 230 that converts linear actuation of the rod 214 into rotation of the spindle 204.

Similarly, the hydraulic line 208 is coupled to a cylinder 220 to rotate the spindle 204. The cylinder 220 includes a diaphragm 222 that is coupled to a rod 224. The diaphragm 222 moves back and forth within the cylinder to rotate the spindle 204. The rod 224 is coupled to the gear system 230 that converts linear actuation of the rod 214 into rotation of the spindle 204.

Referring back to FIG. 2, the handle 42 includes one or more actuator(s) 202 associated with a control interface of the handle 42, e.g., lever or trigger 42a. The actuator 202 for the handle 42 may include a spindle 204 to rotate the respective control interface or the gear system 230 (FIG. 3) may linearly actuate a respective control interface, e.g., trigger 42a. It will be appreciated that each actuator 202 of the handle 42 includes a pneumatic line and a hydraulic line similar to pneumatic and hydraulic lines 206, 208.

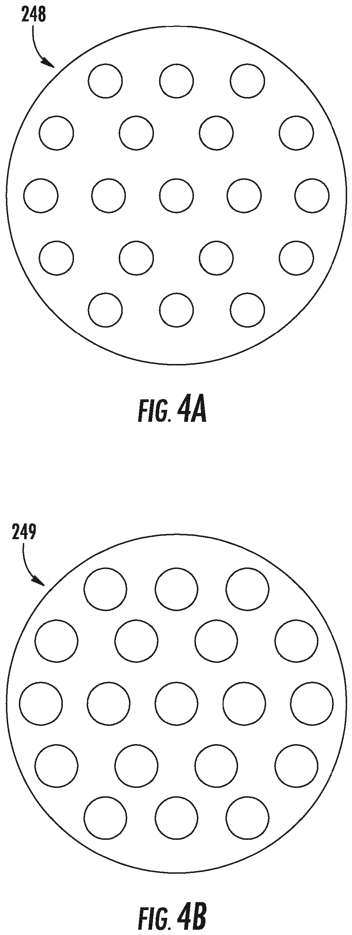

With continued reference to FIG. 2, the pneumatic and hydraulic lines 206, 208 of each actuator 202 extend from the respective joint or control interface, e.g., fourth axis of rotation A₄, to a master manifold 240 of the master user console 40. The master manifold 240 includes a connection for each of the pneumatic and hydraulic lines 206, 208 from the master user console 40 to provide a single point of connection for each of the pneumatic and hydraulic lines 206, 208 off of the master user console 40. It is contemplated that each arm 43, 43' and/or the foot pedals 60 may include an arm or pedal manifold 243, 243', 246 that provides a single point of connection for the respective arm 43, 43' or foot pedals 60 to reduce the number of loose lines extending between a particular arm 43, 43' or foot pedals 60 and the master manifold 240. It will be appreciated that each arm or pedal manifold 243, 243', 246 is coupled to the master manifold 240 by a pneumatic manifold interconnect line 248 and a hydraulic manifold interconnect line 249. Each of the pneumatic and hydraulic manifold interconnect lines 248, 249 includes a discreet internal channel for each of the pneumatic and hydraulic lines, e.g., pneumatic and hydraulic lines 206, 208, that enter the respective arm or pedal manifold 243, 243', 246 as shown in FIGS. 4A and 4B. While the pneumatic and hydraulic manifold interconnect lines 248, 249 are shown as a single line with a single connection to the master manifold 240 and the respective arm or pedal manifold 243, 243', 246, the pneumatic and hydraulic manifold interconnect lines 248, 249 may be a bundle of lines with a separate connector for each line.

Referring briefly back to FIG. 1, the master manifold 240 is coupled to a slave manifold 260 by a pneumatic console interconnect line 256 and a hydraulic console interconnect line 258. The pneumatic and hydraulic console interconnect lines 256, 258 include a discreet channel for each of the pneumatic and hydraulic lines, e.g., pneumatic and hydraulic lines 206, 208, that are coupled to the master manifold 240. While the pneumatic and hydraulic console interconnect lines 256, 258 are shown as a single line with a single connection to each of the master and slave manifolds 240, 260, the pneumatic and hydraulic console interconnect lines 256, 258 may be a bundle of lines with a separate connector for each line or group of lines, e.g., a separate connector for an arm group of lines.

The slave user console 140 includes actuators 202 at joints and control interfaces of the first and second arms 143, 143' and pedals 160 that correspond to the actuator 202 of the master user console 40. The shared control system 200 interconnects the pneumatic and hydraulic lines from each of the actuators 202 of the master user console 40 with a corresponding actuator 202 of the slave user console 140 such that as the joint or the control interface of the master user console 40 is manipulated, the shared control system 200 manipulates the corresponding joint of the slave user console 140 in a similar manner. Specifically, as the joint or the control interface of the master user console 40 back drives the associated actuator 202, the corresponding joint or control interface of the slave user console 140 drives the corresponding actuator 202 such that the corresponding joint or control interface of the slave user console 140 mirrors the movement of the joint or the control interface of the master user console 40. It will be appreciated that movement of a joint or control interface of the slave user console 140 may be mirrored to a joint or control interface of the master user console 40 in a similar manner.

By mirroring movements between the master user console 40 and the slave user console 140, movements of the surgical robot 10 (FIG. 1) can be controlled by either of the master or slave user consoles 40, 140 simultaneously. In addition, movements of one of the user consoles, e.g., master user console 40, may be controlled by a teaching clinician such that a learning clinician can experience movements through the other one of the user console, e.g., slave user console 140. Similarly, a learning clinician can use one of the user consoles, e.g., slave user console 140, to perform a surgical procedure with the surgical robot 10 while a teaching clinician observes the surgical procedure from the other user console, e.g., master user console 40, to provide guidance and/or physical input to assist the learning clinician.

As detailed above, the master and slave user consoles 40, 140 may be used with a surgical simulator instead of a surgical robot. When the master and slave user consoles 40, 140 are used with a surgical simulator, the teaching and learning clinicians can perfect a skill or an entire surgical procedure without acting on a patient.

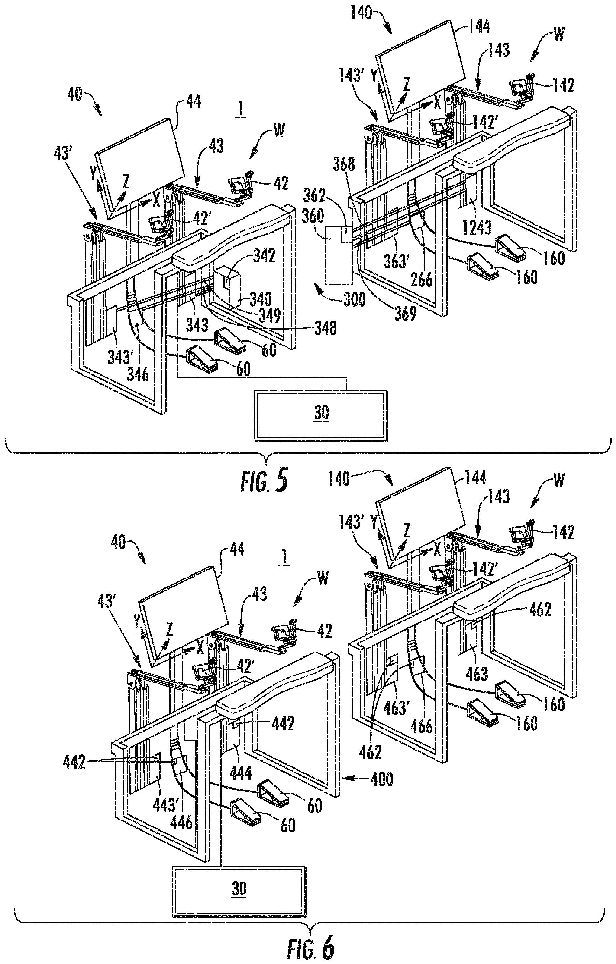

Referring now to FIG. 5, another shared control system 300 is disclosed in accordance with the present disclosure. The shared control system 300 includes the actuators 202 (FIG. 2) and the pneumatic and hydraulic lines associated with each of the actuators 202 of the master and slave user consoles 40, 140, e.g., pneumatic and hydraulic lines 206, 208. The shared control system 300 includes a master manifold 340 and a slave manifold 360. The master manifold 340 and the slave manifold 360 receive the pneumatic and hydraulic lines from the actuators 202 in a similar manner to the master and slave manifolds 240, 260 detailed above.

The master manifold 340 and the slave manifold 360 include a pneumatic fluid actuator 306 associated with each pneumatic line, e.g., pneumatic line 206, and a hydraulic fluid actuator 308 associated with each hydraulic line 208. Each of the pneumatic and hydraulic fluid actuators 306, 308 are configured to remotely drive the associated actuator 202 and be back driven by the associated actuator 202. Each pneumatic and hydraulic fluid actuator 306, 308 includes a sensor 310 configured to determine the position of the diaphragm 212, 222 (FIG. 3) of the associated actuator 202.

The master manifold 340 includes a master controller 342 that is in communication with each of the pneumatic and hydraulic fluid actuators 306, 308 of the master manifold 340 and each of the sensors 310. Similarly the slave manifold 360 includes a slave controller 362 that is in communication with each of the pneumatic and hydraulic fluid actuators 306, 308 of the slave manifold 340. The master controller 342 is in communication with the slave controller 362 to control the corresponding pneumatic and hydraulic fluid actuators 306, 308 of the respective manifold 340, 360 such that the corresponding actuators 202 are mirrored between the master and slave user interfaces 40, 140.

The master and slave controllers 342, 362 may be in direct electrical communication with one another, e.g., be directly wired to one another, or may be in wireless communication with one another. Additionally or alternatively, the master and slave controllers 342, 362 may be in communication, either directly or wirelessly, with the processing unit 30 (FIG. 1). Each of the controllers 342, 362 converts the positions of the diaphragms 212, 222 associated with the pneumatic and hydraulic lines 206, 208 to electrical signals and transmits the positions to the controller 342, 362 of a corresponding manifold 340, 360 which may reduce the need for multiple pneumatic and hydraulic interconnect lines to run between manifolds and/or the user consoles. The reduction in interconnect lines may reduce the cost of the robotic surgical system. In addition, the reduction in interconnect lines may reduce the number of lines within the surgical space and increase the safety and/or the flexibility of the robotic surgical system.

With reference to FIG. 6, another shared control system 400 is disclosed in accordance with the present disclosure. The shared control system 400 includes the actuators 202 and the pneumatic and hydraulic lines associated with each of the actuators 202 of the master and slave user consoles 40, 140, e.g., pneumatic and hydraulic lines 206, 208. The shared control system 400 includes master arm manifolds 443, 443', a master foot manifold 446, slave arm manifolds 463, 463', and a slave foot manifold 466. The master manifolds 443,443', 446 and slave manifolds 463, 463', 466 receive the pneumatic and hydraulic lines from the actuators 202 in a similar manner to the master arm and foot manifolds 243, 243', 246 and the slave manifolds 463, 463', 466 receive the pneumatic and hydraulic lines form the actuators 202 in a similar manner to the slave arm and foot manifolds 263, 263', 266 detailed above.

Each of the master arm and foot manifolds 443, 443', 446 is similar to the master manifold 340 detailed above with a pneumatic and hydraulic fluid actuator 406, 408 associated with each of the pneumatic and hydraulic lines 206, 208, respectively. In addition, each of the slave arm and foot manifolds 463, 463', 466 is similar to the slave manifold 360 detailed above with a pneumatic and hydraulic fluid actuator 406, 408 associated with each of the pneumatic and hydraulic lines 206, 208, respectively.

Each of the master and slave arm and foot manifolds 443, 443', 446, 463, 463', 466 includes a controller 442, 462 that is in communication with each of the pneumatic and fluid actuators 406, 408 and sensors 410 of the respective manifold 443, 443', 446, 463, 463', 466. The controller 442 of the master arm manifold 443 is in communication with the controller 462 of the slave arm manifold 463, the controller 442 of the master arm manifold 443' is in communication with the controller 462 of the slave arm manifold 463', and the controller 442 of the master foot manifold 446 is in communication with the controller 462 of the slave foot manifold 466 such that the actuators 202 of the slave user console 140 mirror the corresponding actuators 202 of the master user console 40 and vice versa as detailed above.

The controllers 442, 462 may be in wired or wireless communication with one another and/or with the processing unit 30 (FIG. 1). Each of the controllers 442, 462 converts the positions of the diaphragms 212, 222 associated with the pneumatic and hydraulic lines 206, 208 to electrical signals and transmits the positions to the controller 442, 462 of a corresponding manifold 443, 443', 446, 463, 463', 466 may reduce the need for multiple pneumatic and hydraulic interconnect lines to run between manifolds and/or the user consoles. The reduction in interconnect lines may reduce the cost of the robotic surgical system. In addition, the reduction in interconnect lines may reduce the number of lines within the surgical space and increase the safety and/or the flexibility of the robotic surgical system.

The shared control systems 300 and 400 may be used with a programed surgical technique or procedure to teach the technique or the procedure to a clinician. The processing unit 30 may be preprogrammed with one or more surgical techniques or procedures such that a clinician interfacing with one of the master or slave user consoles 40, 140 may select a preprogramed technique or procedure. The processing unit 30 then transmits the movements of the technique or the procedure to the manifold or manifold(s), e.g., master manifold 340, of the user console while transmitting images of the simulated procedure to the displays associated with the user consoles, e.g., displays 44, 144 (FIG. 1).

The shared control systems 300 and 400 may be used to program a surgical technique or procedure such that the technique or produce can be taught to clinicians as detailed above. Specifically, when being used during a simulated or actual surgical procedure, a clinician may select a recording option such that the processing unit 30 captures images from the display, e.g., display 44, and positions of the actuators 202 from the manifold(s), e.g., master manifold 340, during a simulated or actual surgical procedure such that the images and positions of the actuators 202 can be transmitted as a preprogramed technique or procedure as detailed above.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

## Claims

1. A dual user console for a robotic surgical system, the dual user console comprising:
a first user console (40) including:
a first arm (43) having a first joint; and
a first actuator (202) disposed at the first joint; and
a second user console (140) including:
a second arm (143) having a second joint; and
a second actuator (202) disposed at the second joint,
wherein movements of the first arm (43) about the first joint are mirrored to movements of the second arm (143) about the second joint and movements of the second arm about the second joint are mirrored to movements of the first arm about the first joint,
**characterized in that**:
the first user console (40) includes a first pneumatic cylinder (210) and a first hydraulic cylinder (220), each of the first pneumatic and hydraulic cylinders configured to actuate the first arm (43) about the first joint and to be back driven by movement of the first arm about the first joint;
the first user console (40) further includes a first manifold (240, 340, 443) coupled to the first pneumatic cylinder (210) and the first hydraulic cylinder (220);
the second user console (140) includes a second pneumatic cylinder (210) and a second hydraulic cylinder (220), each of the second pneumatic and hydraulic cylinders configured to actuate the second arm (143) about the second joint and to be back driven by movement of the second arm about the second joint; and
the second user console (140) further includes a second manifold (260, 360, 463) coupled to the second pneumatic cylinder and the second hydraulic cylinder,
wherein the first manifold (240, 340, 443) is in communication with the second manifold (260, 360, 463) such that said movements of the first arm (43) about the first joint are mirrored to movements of the second arm (143) about the second joint and said movements of the second arm about the second joint are mirrored to movements of the first arm about the first joint.

2. The dual user console according to claim 1, wherein the first user console (40) includes a first pneumatic line (206) and a first hydraulic line (208), the first pneumatic line (206) having a first end coupled to the first pneumatic cylinder (210) and a second end coupled to the first manifold (240, 340, 443), the first hydraulic line (208) having a first end coupled to the first hydraulic cylinder (220) and a second end coupled to the first manifold (240, 340, 443).

3. The dual user console according to claim 1, or 2 wherein the first manifold (443) is disposed on the first arm (43).

4. The dual user console according to claim 1 or 2 wherein the first manifold (240, 340) is a first console manifold (240) that is coupled to the first pneumatic cylinder (210) and the first hydraulic cylinder (220) through a first arm manifold (243, 343).

5. The dual user console according to claim 4, wherein the first arm manifold (243, 343) is disposed on the first arm (43).

6. The dual user console according to claim 4, further comprising a pneumatic manifold interconnect (248) extending between the first console manifold (240, 340) and the first arm manifold (243, 343), and a hydraulic manifold interconnect (249) extending between the first console manifold (240, 340) and the first arm manifold (243, 343).

7. The dual user console according to any preceding claim, further comprising a pneumatic console interconnect (256) extending between the first manifold (240) and the second manifold (260), and a hydraulic console interconnect (258) extending between the first manifold and the second manifold, the pneumatic console interconnect (256) pneumatically coupling the first and second manifolds, and the hydraulic console interconnect (258) hydraulically coupling the first and second manifolds (240, 260).

8. The dual user console according to any preceding claim, wherein the first manifold (340, 443) is in wired or wireless communication with the second manifold (360, 463), the first manifold configured to transmit positions of the first pneumatic and first hydraulic cylinders (210, 220) to the second manifold such that the second manifold actuates the second pneumatic and second hydraulic cylinders to mirror the positions of first pneumatic and hydraulic cylinders.

9. The dual user console according to claim 8, wherein the second manifold (360, 463) is configured to transmit positions of the second pneumatic and hydraulic cylinders to the first manifold (340, 443) such that the first manifold actuates the first pneumatic and hydraulic cylinders (210, 220) to mirror the positions of the second pneumatic and hydraulic cylinders.

10. The dual user console according to any preceding claim , wherein the first user console (40) includes a third arm having a third joint and a third actuator disposed at the third joint, wherein the second user console (140) includes a fourth arm having a fourth joint and a fourth actuator disposed at the fourth joint, wherein the third actuator is coupled to the first manifold (240, 340, 443) and the fourth actuator is coupled to the second manifold (260, 360, 463), and wherein the first manifold is in communication with the second manifold such that movements of the third arm about the third joint are mirrored to movements of the fourth arm about the fourth joint and movements of the fourth arm about the fourth joint are mirrored to movements of the third arm about the third joint.

11. The dual user console according to any preceding claim, wherein the dual console forms part of a robotic surgical system (1) further comprising:
a processing unit (30); and
a surgical robot (10) having a first robot arm (12);
wherein the first user console (40) is in communication with the processing unit (30) and configured to transmit input signals to the processing unit, and the processing unit is configured to transmit control signals to the surgical robot in response to the input signals such that the surgical robot manipulates the first robot arm (12) in response to the control signals.

12. A method of controlling a surgical robot (1) or a surgical simulator comprising a dual user console according to any of claims 1 to 11, the method comprising:
manipulating the first arm (43) of the first user console (40) about the first joint such that the first actuator (202) associated with the first joint transmits first pneumatic and hydraulic signals to the second actuator associated with the second joint of the second arm of the second user console to actuate the second arm (143) to mirror the manipulation of the first arm; and
manipulating the second arm (143) of the second user console (140) about the second joint such that the second actuator transmits second pneumatic and hydraulic signals to the first actuator associated with the first joint to actuate the first arm (43) to mirror the manipulation of the second arm.

13. The method according to claim 12, wherein manipulating the first arm (43) includes transmitting the first pneumatic and hydraulic signals to the first manifold (240, 340, 443) of the first user console (40) which transmits the first pneumatic and hydraulic signals to the second manifold (260, 360, 363) of the second user console (140).

14. The method according to claim 13, wherein the first manifold (340, 443) transmitting the first pneumatic and hydraulic signals to the second manifold (360, 363) includes via wire or wirelessly transmitting the first pneumatic and hydraulic signals.

15. The method according to claim 14, further comprising selecting a preprogramed surgical technique such that a processing unit of the surgical robot (1) or the surgical simulator transmits control signals to the first manifold (240, 340, 443), the first manifold transmitting pneumatic and hydraulic signals to the first actuator (202) in response to the control signals such that the first actuator (202) actuates the first arm (43) about the first joint.

## Patentansprüche

1. Doppelbenutzerkonsole für ein chirurgisches Robotersystem, die Doppelbenutzerkonsole umfassend:
eine erste Benutzerkonsole (40), einschließlich:
eines ersten Arms (43), der ein erstes Gelenk aufweist; und
eines ersten Betätigungselements (202), das an dem ersten Gelenk angeordnet ist; und
eine zweite Benutzerkonsole (140), einschließlich:
eines zweiten Arms (143), der ein zweites Gelenk aufweist; und
eines zweiten Betätigungselements (202), das an dem zweiten Gelenk angeordnet ist,
wobei Bewegungen des ersten Arms (43) um das erste Gelenk herum zu Bewegungen des zweiten Arms (143) um das zweite Gelenk herum gespiegelt werden und Bewegungen des zweiten Arms um das zweite Gelenk herum zu Bewegungen des ersten Arms um das erste Gelenk herum gespiegelt werden,
**dadurch gekennzeichnet, dass:**
die erste Benutzerkonsole (40) einen ersten Pneumatikzylinder (210) und einen ersten Hydraulikzylinder (220) einschließt, wobei jeder der ersten Pneumatik- und Hydraulikzylinder konfiguriert ist, um den ersten Arm (43) um das erste Gelenk herum zu betätigen und durch Bewegung des ersten Arms um das erste Gelenk herum rückwärts angetrieben zu werden;
die erste Benutzerkonsole (40) ferner einen ersten Verteiler (240, 340, 443) einschließt, der mit dem ersten Pneumatikzylinder (210) und dem ersten Hydraulikzylinder (220) gekoppelt ist;
die zweite Benutzerkonsole (140) einen zweiten Pneumatikzylinder (210) und einen zweiten Hydraulikzylinder (220) einschließt, wobei jeder der zweiten Pneumatik- und Hydraulikzylinder konfiguriert ist, um den zweiten Arm (143) um das zweite Gelenk herum zu betätigen und durch Bewegung des zweiten Arms um das zweite Gelenk herum rückwärts angetrieben zu werden; und
die zweite Benutzerkonsole (140) ferner einen zweiten Verteiler (260, 360, 463) einschließt, der mit dem zweiten Pneumatikzylinder und dem zweiten Hydraulikzylinder gekoppelt ist,
wobei der erste Verteiler (240, 340, 443) mit dem zweiten Verteiler (260, 360, 463) derart in Kommunikation steht, dass die Bewegungen des ersten Arms (43) um das erste Gelenk herum zu Bewegungen des zweiten Arms (143) um das zweite Gelenk herum gespiegelt werden und die Bewegungen des zweiten Arms um das zweite Gelenk herum zu Bewegungen des ersten Arms um das erste Gelenk herum gespiegelt werden.

2. Doppelbenutzerkonsole nach Anspruch 1, wobei die erste Benutzerkonsole (40) eine erste Pneumatikleitung (206) und eine erste Hydraulikleitung (208) einschließt, wobei die erste Pneumatikleitung (206) ein erstes Ende, das mit dem ersten Pneumatikzylinder (210) gekoppelt ist, und ein zweites Ende aufweist, das mit dem ersten Verteiler (240, 340, 443) gekoppelt ist, wobei die erste Hydraulikleitung (208) ein erstes Ende, das mit dem ersten Hydraulikzylinder (220) gekoppelt ist, und ein zweites Ende aufweist, das mit dem ersten Verteiler (240, 340, 443) gekoppelt ist.

3. Doppelbenutzerkonsole nach Anspruch 1 oder 2, wobei der erste Verteiler (443) an dem ersten Arm (43) angeordnet ist.

4. Doppelbenutzerkonsole nach Anspruch 1 oder 2, wobei der erste Verteiler (240, 340) ein erster Konsolenverteiler (240) ist, der mit dem ersten Pneumatikzylinder (210) und dem ersten Hydraulikzylinder (220) über einen ersten Armverteiler (243, 343) gekoppelt ist.

5. Doppelbenutzerkonsole nach Anspruch 4, wobei der erste Armverteiler (243, 343) an dem ersten Arm (43) angeordnet ist.

6. Doppelbenutzerkonsole nach Anspruch 4, ferner umfassend eine Pneumatikverteilerverbindung (248), die sich zwischen dem ersten Konsolenverteiler (240, 340) und dem ersten Armverteiler (243, 343) erstreckt, und eine Hydraulikverteilerverbindung (249), die sich zwischen dem ersten Konsolenverteiler (240, 340) und dem ersten Armverteiler (243, 343) erstreckt.

7. Doppelbenutzerkonsole nach einem der vorstehenden Ansprüche, ferner umfassend eine Pneumatikkonsolenverbindung (256), die sich zwischen dem ersten Verteiler (240) und dem zweiten Verteiler (260) erstreckt, und eine Hydraulikkonsolenverbindung (258), die sich zwischen dem ersten Verteiler und dem zweiten Verteiler erstreckt, wobei die Pneumatikkonsolenverbindung (256) den ersten und den zweiten Verteiler pneumatisch koppelt, und die Hydraulikkonsolenverbindung (258) den ersten und den zweiten Verteiler (240, 260) hydraulisch koppelt.

8. Doppelbenutzerkonsole nach einem der vorstehenden Ansprüche, wobei der erste Verteiler (340, 443) in einer drahtgebundenen oder drahtlosen Kommunikation mit dem zweiten Verteiler (360, 463) steht, wobei der erste Verteiler konfiguriert ist, um Positionen des ersten Pneumatik- und ersten Hydraulikzylinders (210, 220) an den zweiten Verteiler derart zu übertragen, dass der zweite Verteiler die zweiten Pneumatik- und zweiten Hydraulikzylinder betätigt, um die Positionen der ersten Pneumatik- und Hydraulikzylinder zu spiegeln.

9. Doppelbenutzerkonsole nach Anspruch 8, wobei der zweite Verteiler (360, 463) konfiguriert ist, um Positionen der zweiten Pneumatik- und Hydraulikzylinder an den ersten Verteiler (340, 443) derart zu übertragen, dass der erste Verteiler die ersten Pneumatik- und Hydraulikzylinder (210, 220) betätigt, um die Positionen der zweiten Pneumatik- und Hydraulikzylinder zu spiegeln.

10. Doppelbenutzerkonsole nach einem der vorstehenden Ansprüche, wobei die erste Benutzerkonsole (40) einen dritten Arm einschließt, der ein drittes Gelenk und ein drittes Betätigungselement aufweist, das an dem dritten Gelenk angeordnet ist, wobei die zweite Benutzerkonsole (140) einen vierten Arm einschließt, der ein viertes Gelenk und ein viertes Betätigungselement aufweist, das an dem vierten Gelenk angeordnet ist, wobei das dritte Betätigungselement mit dem ersten Verteiler (240, 340, 443) gekoppelt ist und das vierte Betätigungselement mit dem zweiten Verteiler (260, 360, 463) gekoppelt ist, und wobei der erste Verteiler mit dem zweiten Verteiler derart in Kommunikation steht, dass Bewegungen des dritten Arms um das dritte Gelenk herum zu Bewegungen des vierten Arms um das vierte Gelenk herum gespiegelt werden und Bewegungen des vierten Arms um das vierte Gelenk herum zu Bewegungen des dritten Arms um das dritte Gelenk herum gespiegelt werden.

11. Doppelbenutzerkonsole nach einem der vorstehenden Ansprüche, wobei die Doppelkonsole einen Teil eines chirurgischen Robotersystems (1) ausbildet, ferner umfassend:
eine Verarbeitungseinheit (30); und
einen chirurgischen Roboter (10), der einen ersten Roboterarm (12) aufweist;
wobei die erste Benutzerkonsole (40) mit der Verarbeitungseinheit (30) in Kommunikation steht und konfiguriert ist, um Eingangssignale an die Verarbeitungseinheit zu übertragen, und die Verarbeitungseinheit konfiguriert ist, um Steuersignale an den chirurgischen Roboter als Reaktion auf die Eingangssignale derart zu übertragen, dass der chirurgische Roboter den ersten Roboterarm (12) als Reaktion auf die Steuersignale manipuliert.

12. Verfahren zum Steuern eines chirurgischen Roboters (1) oder eines chirurgischen Simulators, umfassend eine Doppelbenutzerkonsole nach einem der Ansprüche 1 bis 11, das Verfahren umfassend:
derartiges Manipulieren des ersten Arms (43) der ersten Benutzerkonsole (40) um das erste Gelenk herum, dass das erste Betätigungselement (202), das dem ersten Gelenk zugeordnet ist, erste Pneumatik- und Hydrauliksignale an das zweite Betätigungselement überträgt, das dem zweiten Gelenk des zweiten Arms der zweiten Benutzerkonsole zugeordnet ist, um den zweiten Arm (143) zu betätigen, um die Manipulation des ersten Arms zu spiegeln; und
derartiges Manipulieren des zweiten Arms (143) der zweiten Benutzerkonsole (140) um das zweite Gelenk herum, dass das zweite Betätigungselement zweite Pneumatik- und Hydrauliksignale an das erste Betätigungselement überträgt, das dem ersten Gelenk zugeordnet ist, um den ersten Arm (43) zu betätigen, um die Manipulation des zweiten Arms zu spiegeln.

13. Verfahren nach Anspruch 12, wobei das Manipulieren des ersten Arms (43) das Übertragen der ersten Pneumatik- und Hydrauliksignale an den ersten Verteiler (240, 340, 443) der ersten Benutzerkonsole (40) einschließt, der die ersten Pneumatik- und Hydrauliksignale an den zweiten Verteiler (260, 360, 363) der zweiten Benutzerkonsole (140) überträgt.

14. Verfahren nach Anspruch 13, wobei der erste Verteiler (340, 443), der die ersten Pneumatik- und Hydrauliksignale an den zweiten Verteiler (360, 363) überträgt, das drahtgebundene oder drahtlose Übertragen der ersten Pneumatik- und Hydrauliksignale einschließt.

15. Verfahren nach Anspruch 14, ferner umfassend ein derartiges Auswählen einer vorprogrammierten chirurgischen Technik, dass eine Verarbeitungseinheit des chirurgischen Roboters (1) oder des chirurgischen Simulators Steuersignale an den ersten Verteiler (240, 340, 443) überträgt, wobei der erste Verteiler Pneumatik- und Hydrauliksignale an das erste Betätigungselement (202) als Reaktion auf die Steuersignale derart überträgt, dass das erste Betätigungselement (202) den ersten Arm (43) um das erste Gelenk herum betätigt.

## Revendications

1. Console double utilisateur destinée à un système chirurgical robotique, la console double utilisateur comprenant :
une première console utilisateur (40) comportant :
un premier bras (43) ayant une première articulation ; et
un premier actionneur (202) disposé au niveau de la première articulation ; et une seconde console utilisateur (140) comportant :
un deuxième bras (143) ayant une deuxième articulation ; et
un deuxième actionneur (202) disposé au niveau de la deuxième articulation, dans laquelle des mouvements du premier bras (43) autour de la première articulation se reflètent dans des mouvements du deuxième bras (143) autour de la deuxième articulation et des mouvements du deuxième bras autour de la deuxième articulation se reflètent dans des mouvements du premier bras autour de la première articulation,
**caractérisée en ce que** :
la première console utilisateur (40) comporte un premier vérin pneumatique (210) et un premier vérin hydraulique (220), chacun des premiers vérins pneumatique et
hydraulique étant conçu pour actionner le premier bras (43) autour de la première articulation et pour être entraîné vers l'arrière par un mouvement du premier bras autour de la première articulation ;
la première console utilisateur (40) comporte en outre un premier collecteur (240, 340, 443) accouplé au premier vérin pneumatique (210) et au premier vérin hydraulique (220) ;
la seconde console utilisateur (140) comporte un second vérin pneumatique (210) et un second vérin hydraulique (220), chacun des seconds vérins pneumatique et
hydraulique étant conçu pour actionner le deuxième bras (143) autour de la deuxième articulation et pour être entraîné vers l'arrière par un mouvement du deuxième bras autour de la deuxième articulation ; et
la seconde console utilisateur (140) comporte en outre un second collecteur (260, 360, 463) accouplé au second vérin pneumatique et au second vérin hydraulique,
dans laquelle le premier collecteur (240, 340, 443) est en communication avec le second collecteur (260, 360, 463) de telle sorte que lesdits mouvements du premier bras (43) autour de la première articulation se reflètent dans des mouvements du deuxième bras (143) autour de la deuxième articulation et lesdits mouvements du deuxième bras autour de la deuxième articulation se reflètent dans des mouvements du premier bras autour de la première articulation.

2. Console double utilisateur selon la revendication 1, dans laquelle la première console utilisateur (40) comporte une première conduite pneumatique (206) et une première conduite hydraulique (208), la première conduite pneumatique (206) ayant une première extrémité accouplée au premier vérin pneumatique (210) et une seconde extrémité accouplée au premier collecteur (240, 340, 443), la première conduite hydraulique (208) ayant une première extrémité accouplée au premier vérin hydraulique (220) et une seconde extrémité accouplée au premier collecteur (240, 340, 443).

3. Console double utilisateur selon la revendication 1 ou 2, dans laquelle le premier collecteur (443) est disposé sur le premier bras (43).

4. Console double utilisateur selon la revendication 1 ou 2, dans laquelle le premier collecteur (240, 340) est un collecteur de la première console (240) qui est accouplé au premier vérin pneumatique (210) et au premier vérin hydraulique (220) par l'intermédiaire d'un collecteur du premier bras (243, 343).

5. Console double utilisateur selon la revendication 4, dans laquelle le collecteur du premier bras (243, 343) est disposé sur le premier bras (43).

6. Console double utilisateur selon la revendication 4, comprenant en outre un raccord de collecteur pneumatique (248) s'étendant entre le collecteur de la première console (240, 340) et le collecteur du premier bras (243, 343), et un raccord de collecteur hydraulique (249) s'étendant entre le collecteur de la première console (240, 340) et le collecteur du premier bras (243, 343).

7. Console utilisateur double selon l'une quelconque des revendications précédentes, comprenant en outre un raccord de console pneumatique (256) s'étendant entre le premier collecteur (240) et le second collecteur (260), et un raccord de console hydraulique (258) s'étendant entre le premier collecteur et le second collecteur, le raccord de console pneumatique (256) accouplant pneumatiquement les premier et second collecteurs, et le raccord de console hydraulique (258) accouplant hydrauliquement les premier et second collecteurs (240, 260).

8. Console double utilisateur selon l'une quelconque des revendications précédentes, dans laquelle le premier collecteur (340, 443) est en communication par fil ou sans fil avec le second collecteur (360, 463), le premier collecteur étant conçu pour transmettre les positions des premiers vérins pneumatique et hydraulique (210, 220) au second collecteur de sorte que le second collecteur actionne les seconds vérins pneumatique et hydraulique pour refléter les positions des premiers vérins pneumatique et hydraulique.

9. Console double utilisateur selon la revendication 8, dans laquelle le second collecteur (360, 463) est conçu pour transmettre les positions des seconds vérins pneumatique et hydraulique au premier collecteur (340, 443) de telle sorte que le premier collecteur actionne les premiers vérins pneumatique et hydraulique (210, 220) pour refléter les positions des seconds vérins pneumatique et hydraulique.

10. Console double utilisateur selon l'une quelconque des revendications précédentes, dans laquelle la première console utilisateur (40) comporte un troisième bras ayant une troisième articulation et un troisième actionneur disposé au niveau de la troisième articulation, la seconde console utilisateur (140) comportant un quatrième bras ayant une quatrième articulation et un quatrième actionneur disposé au niveau de la quatrième articulation, le troisième actionneur étant accouplé au premier collecteur (240, 340, 443) et le quatrième actionneur étant accouplé au second collecteur (260, 360, 463), et le premier collecteur étant en communication avec le second collecteur de sorte que des mouvements du troisième bras autour de la troisième articulation se reflètent dans des mouvements du quatrième bras autour de la quatrième articulation et les mouvements du quatrième bras autour de la quatrième articulation se reflètent dans des mouvements du troisième bras autour de la troisième articulation.

11. Console double utilisateur selon l'une quelconque des revendications précédentes, dans laquelle la console double fait partie d'un système chirurgical robotique (1) comprenant en outre :
une unité de traitement (30) ; et
un robot chirurgical (10) ayant un premier bras de robot (12) ;
dans laquelle la première console utilisateur (40) est en communication avec l'unité de traitement (30) et configurée pour transmettre des signaux d'entrée à l'unité de traitement, et l'unité de traitement est configurée pour transmettre des signaux de commande au robot chirurgical en réponse aux signaux d'entrée de telle sorte que le robot chirurgical manipule le premier bras de robot (12) en réponse aux signaux de commande.

12. Procédé de commande d'un robot chirurgical (1) ou d'un simulateur chirurgical comprenant une console double utilisateur selon l'une quelconque des revendications 1 à 11, le procédé comprenant :
la manipulation du premier bras (43) de la première console utilisateur (40) autour de la première articulation de sorte que le premier actionneur (202) associé à la première articulation transmette des premiers signaux pneumatiques et hydrauliques au deuxième actionneur associé à la deuxième articulation du deuxième bras de la seconde console utilisateur pour actionner le deuxième bras (143) afin de refléter la manipulation du premier bras ; et
la manipulation du deuxième bras (143) de la seconde console utilisateur (140) autour de la deuxième articulation de sorte que le deuxième actionneur transmette des seconds signaux pneumatiques et hydrauliques au premier actionneur associé à la première articulation pour actionner le premier bras (43) afin de refléter la manipulation du deuxième bras.

13. Procédé selon la revendication 12, dans lequel la manipulation du premier bras (43) comporte la transmission des premiers signaux pneumatiques et hydrauliques au premier collecteur (240, 340, 443) de la première console utilisateur (40) qui transmet les premiers signaux pneumatiques et hydrauliques au second collecteur (260, 360, 363) de la seconde console utilisateur (140).

14. Procédé selon la revendication 13, dans lequel le premier collecteur (340, 443) transmettant les premiers signaux pneumatiques et hydrauliques au second collecteur (360, 363) comporte la transmission par fil ou sans fil des premiers signaux pneumatiques et hydrauliques.

15. Procédé selon la revendication 14, comprenant en outre la sélection d'une technique chirurgicale préprogrammée de telle sorte qu'une unité de traitement du robot chirurgical (1) ou du simulateur chirurgical transmette des signaux de commande au premier collecteur (240, 340, 443), le premier collecteur transmettant des signaux pneumatiques et hydrauliques au premier actionneur (202) en réponse aux signaux de commande de telle sorte que le premier actionneur (202) actionne le premier bras (43) autour de la première articulation.
